# EUROPEAN PATENT SPECIFICATION

(11) **EP 4 355 254 B1**
(45) Date of publication and mention of the grant of the patent: **25.03.2026**
(21) Application number: 22738049.0
(22) Date of filing: 12.06.2022
(51) Int. Cl.: A61B 46/10, A61B 90/96, A61B 90/00, A61B 34/20

(54) **SYSTEMS FOR DETECTING AND MONITORING A DRAPE CONFIGURATION**
SYSTEME ZUR ERKENNUNG UND ÜBERWACHUNG EINER ABDECKTUCHKONFIGURATION
SYSTÈMES DE DÉTECTION ET DE SURVEILLANCE D'UNE CONFIGURATION DE CHAMP OPÉRATOIRE

(30) Priority: 14.06.2021 US 202163210150 P
(43) Date of publication of application: 24.04.2024
(73) Proprietor: Mazor Robotics Ltd., 3079830 Caesarea (IL)
(72) Inventor: SEEMANN, Ziv, 3079830 Caesarea (IL); SANDELSON, Adi, 3079830 Caesarea (IL); KAGAN, Bat-El, 3079830 Caesarea (IL); RAWLUK, Nicholas J., Louisville, Colorado 80027 (US); PAITEL, Yvan R., Louisville, Colorado 80027 (US)
(74) Representative: Viering, Jentschura & Partner mbB Patent- und Rechtsanwälte
(86) International application number: PCT/IL2022/050623
(87) International publication number: WO 2022/264125

(56) References cited:
- WO-A1-2013/055707
- US-A- 5 653 938
- US-A1- 2019 099 232

## Description

### FIELD

The present technology generally relates to drapes, and relates more particularly to detecting and/or monitoring a configuration of a drape.

### BACKGROUND

Surgical drapes may be used by a surgeon or other medical provider to separate non-sterile components from a sterile environment. Non-sterile components may include, for example, navigation markers, an imaging device, or surgical robots. A surgical drape may shift over time from an initial position due to, for example, movement of the non-sterile component.

From e.g.US 2019 / 099 232 A1 a system is known, comprising a drape, one or more indicia, wherein the one or more indicia is visible when at least a portion of the drape is in a predetermined configuration, one or more sensors configured for sensing the at least one indicia and transmitting information about the at least one indicia to a processor, a memory and the processor.

Further, from e.g. US 5 653 938 B a similar system is known.

### SUMMARY

The present invention provides a system with the features according to claim 1. Further preferred embodiments are described in the dependent claims.

It is to be appreciated that any feature described herein can be claimed in combination with any other feature(s) as described herein, regardless of whether the features come from the same described embodiment.

The details of one or more aspects of the disclosure are set forth in the accompanying drawings and the description below. Other features, objects, and advantages of the techniques described in this disclosure will be apparent from the description and drawings, and from the claims.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X₁-Xₙ, Y₁-Yₘ, and Z₁-Zₒ, the phrase is intended to refer to a single element selected from X, Y, and Z, a combination of elements selected from the same class (e.g., X₁ and X₂) as well as a combination of elements selected from two or more classes (e.g., Y₁ and Zₒ).

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

Numerous additional features and advantages of the present invention will become apparent to those skilled in the art upon consideration of the embodiment descriptions provided hereinbelow.

### BRIEF DESCRIPTION OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure can be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below.
Fig. 1A is a schematic diagram of a system according to at least one embodiment of the present disclosure;
Fig. 1B is a schematic diagram of a component and a portion of a drape of the system of Fig. 1A according to at least one embodiment of the present disclosure;
Fig. 2 is a block diagram of a system according to at least one embodiment of the present disclosure; and
Fig. 3 is a flowchart according to at least one embodiment of the present disclosure.

### DETAILED DESCRIPTION

It should be understood that various aspects disclosed herein may be combined in different combinations than the combinations specifically presented in the description and accompanying drawings. It should also be understood that, depending on the example or embodiment, certain acts or events of any of the processes or methods described herein may be performed in a different sequence, and/or may be added, merged, or left out altogether (e.g., all described acts or events may not be necessary to carry out the disclosed techniques according to different embodiments of the present disclosure). In addition, while certain aspects of this disclosure are described as being performed by a single module or unit for purposes of clarity, it should be understood that the techniques of this disclosure may be performed by a combination of units or modules associated with, for example, a computing device and/or a medical device.

In one or more examples, the described methods, processes, and techniques may be implemented in hardware, software, firmware, or any combination thereof. If implemented in software, the functions may be stored as one or more instructions or code on a computer-readable medium and executed by a hardware-based processing unit. Alternatively or additionally, functions may be implemented using machine learning models, neural networks, artificial neural networks, or combinations thereof (alone or in combination with instructions). Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors (e.g., Intel Core i3, i5, i7, or i9 processors; Intel Celeron processors; Intel Xeon processors; Intel Pentium processors; AMD Ryzen processors; AMD Athlon processors; AMD Phenom processors; Apple A10 or 10X Fusion processors; Apple A11, A12, A12X, A12Z, or A13 Bionic processors; or any other general purpose microprocessors), graphics processing units (e.g., Nvidia GeForce RTX 2000-series processors, Nvidia GeForce RTX 3000-series processors, AMD Radeon RX 5000-series processors, AMD Radeon RX 6000-series processors, or any other graphics processing units), application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

Before any embodiments of the disclosure are explained in detail, it is to be understood that the disclosure is not limited in its application to the details of construction and the arrangement of components set forth in the following description or illustrated in the drawings. The disclosure is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, the present disclosure may use examples to illustrate one or more aspects thereof. Unless explicitly stated otherwise, the use or listing of one or more examples (which may be denoted by "for example," "by way of example," "e.g.," "such as," or similar language) is not intended to and does not limit the scope of the present disclosure.

The terms proximal and distal are used in this disclosure with their conventional medical meanings, proximal being closer to the operator or user of the system, and further from the region of surgical interest in or on the patient, and distal being closer to the region of surgical interest in or on the patient, and further from the operator or user of the system.

When draping a component such as an object or an item, a visible shape of the component can be distorted or obstructed. This optical distortion in shape may affect an accuracy of a navigation system tracking a component such as, for example, light emitting diodes positioned underneath the drape. Draping is conventionally a manually performed task that is sensitive to a user performing the draping and a quality of the drape. If the draping process is executed poorly, the accuracy of the navigation system relying on, for example, light emitting diodes, RADIX, or any reflective item, positioned underneath the drape, may be reduced dramatically. Additionally, certain actions by a robot that is draped may also be cumbersome when the drape is placed or positioned poorly. An advantage of the present disclosure provides for monitoring of the quality of the drape and may be used to aid in notifying a user of a poorly positioned drape.

A navigation camera of a navigation system may, in some instances, have an option of operating as, for example, a "regular" High Definition ("HD") quality camera, which can be used to monitor certain components, objects, or items during an operation or surgical procedure. In other instances, the navigation camera may operate as a camera with sufficient image quality to monitor certain components, objects, or items during an operation or surgical procedure. At least one embodiment of the present disclosure provides for using printed markings or indicia on a drape or below a surface of the drape and monitoring the markings or indicia using the HD camera (or any other imaging device). Information from the imaging device may be used to determine an initial drape condition or configuration. Markings or indicia on the drape itself may also be used to track an accuracy of placement of the drape on selected regions. For example, markings or indicia on the drape may be used to verify that the drape is correctly installed onto a component such as a robotic arm. For example, if the markings or indicia are straight lines, but appear as a zig-zag line, this may indicate that placement of the drape was inaccurate. Another example is if the marking or indicium is a circle and the marking or indicum appears as folded when the drape is installed, which may indicate that placement of the drape was inaccurate. In another example, if the system includes a vacuum in the design of the arm, then the markings or indicia should be verified to have a specific shape before advancing to navigation. In still another example, the markings or indicia may be markings on a robotic joint that can be detected by the HD camera. If the markings or indicia are clear to the HD camera, then this may indicate that the drape is positioned correctly. In another example, the markings or indicia can also include a QR code, barcode, or predefined shape on the drape or on a surface underneath the drape to indicate or verify that a hospital is using a compatible drape - i.e., a system would not advance to navigation until the code is read. The drape can also be disposable.

Embodiments of the present disclosure may improve navigation accuracy and the navigation system's robustness to noise. Embodiments of the present disclosure can also help prepare placement of a drape onto a robotic arm or other equipment.

Embodiments of the present disclosure provide technical solutions to one or more of the problems of (1) determining if a drape is in a predetermined configuration; (2) improving navigational accuracy of components disposed underneath a drape; (3) improving drape positioning and installation; and (4) presenting a clear indication to a user of a drape effect on navigation.

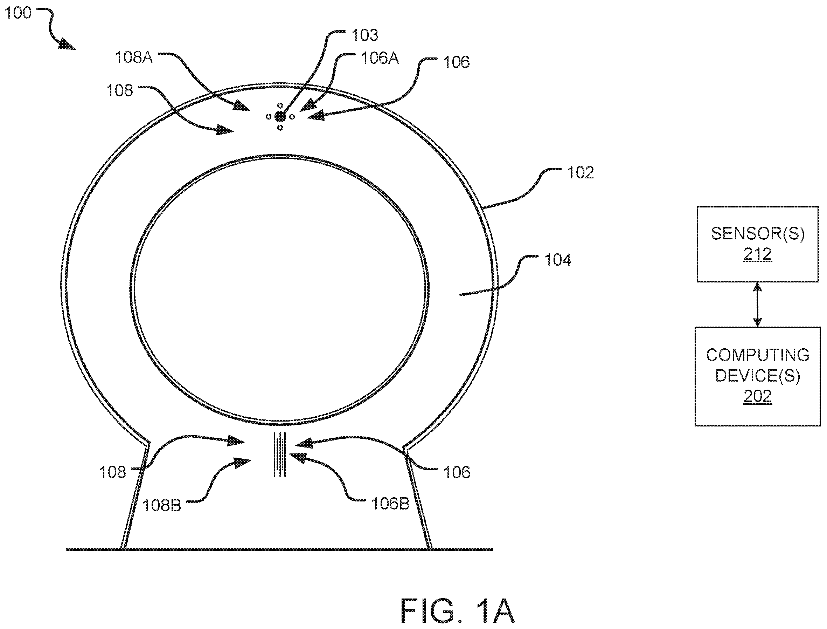

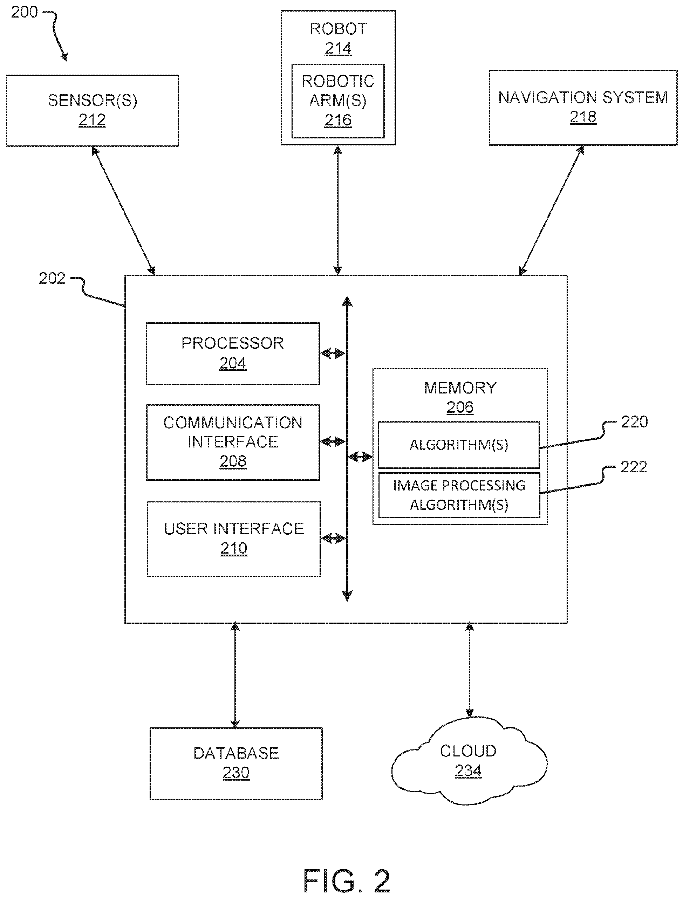

Turning first to Fig. 1A, a drape detection and/or monitoring system 100 according to at least one embodiment of the present disclosure is shown. The system 100 includes a drape 102, one or more sensors 212, and one or more computing devices 202. The drape may be disposable in some embodiments, and reusable in other embodiments. The drape 102 may be positioned over a component 104 such as, for example, an O-arm imaging device. It will be appreciated that the drape 102 may be positioned over any component such as, for example, a robot 214 (shown in Fig. 2), a robotic arm 216 (shown in Fig. 2), any tool, any instrument, any device, any surgical device, or any other component of a surgical environment and/or surgical room.

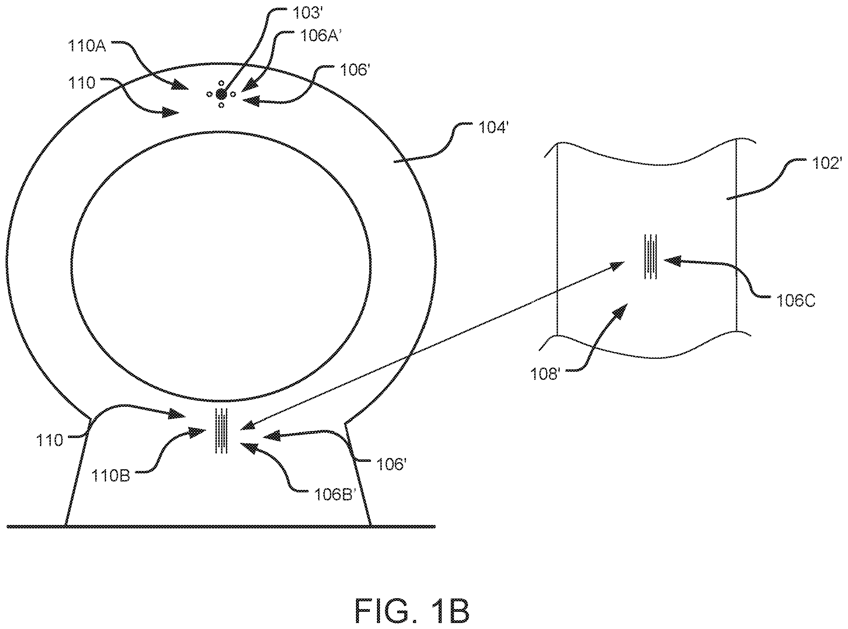

In the illustrated embodiment, at least one indicia 106 is disposed on at least a portion 108 of the drape 102. In other instances, such as the embodiment shown in Fig. 1B, the at least one indicia 106 may be disposed on a portion 110 of the component 104. In some embodiments, when the drape 102 is positioned over the component 104, the at least one indicia 106 may be positioned near at least one navigational marker 103 affixed to, for example, the component 104 (which may be, for example, a surgical device). In embodiments where the at least one indicia 106 is disposed or otherwise integrated with the component 104, the at least one navigational marker 103 may be affixed or positioned near the at least one indicia 106. In some embodiments, the at least one indicia 106 may not be positioned near the at least one marker 103. The at least one navigational marker 103 may comprise one or more active markers, one or more passive markers, or a combination of active and passive markers. The at least one navigational marker 103 may be, for example, light emitting diodes, infrared light emitting diodes, reflective markers, or the like. In some embodiments, the at least one navigational marker 103 and the at least one indicia 106 may be disposed inside the drape 102. In other embodiments, the at least one navigational marker 103 and the at least one indicia 106 may be disposed outside of the drape 102. In still other embodiments, one of the at least one navigational marker 103 and the at least one indicia 106 may be disposed inside the drape 102 and another of the at least one navigational marker 103 and the at least one indicia 106 may be disposed outside of the drape 102.

The at least one indicia 106 may be painted onto, adhered to, or otherwise affixed or integrated with the drape 102 and/or the component 104. In some embodiments, the at least one indicia 106 may comprise one or more active markers such as, for example, infrared light emitting diodes. In other embodiments, the at least one indicia 106 may comprise an optical marker. In still other embodiments, the at least one indicia may comprise at least one optical marker and at least one active marker (such as, for example, an infrared marker). The at least one indicia 106 may be ink printed directly on the drape 102 and/or the component 104, any component of the system 100, and/or any component external to the system 100. In other instances, the at least one indicia 106 may be stickers that are placed on the drape 102 and/or the component 104, on a surface underneath the drape 102, any component of the system 100, and/or any component external to the system 100. In other embodiments, the at least one indicia 106 can be visible ink, ink that is visible in response to being illuminated with light of a certain wavelength, light emitting diodes (e.g., active markers rather than ink), magnetic dots, stickers with ink, combinations thereof, or any other type of marker or indicator. The at least one indicia 106 may comprise, for example, a shape, straight lines, markings (whether active or passive) arranged in a pattern, markings arranged in geometric shapes (e.g., circles, rectangles, squares, triangles, etc.) letters and/or numbers, or a QR code.

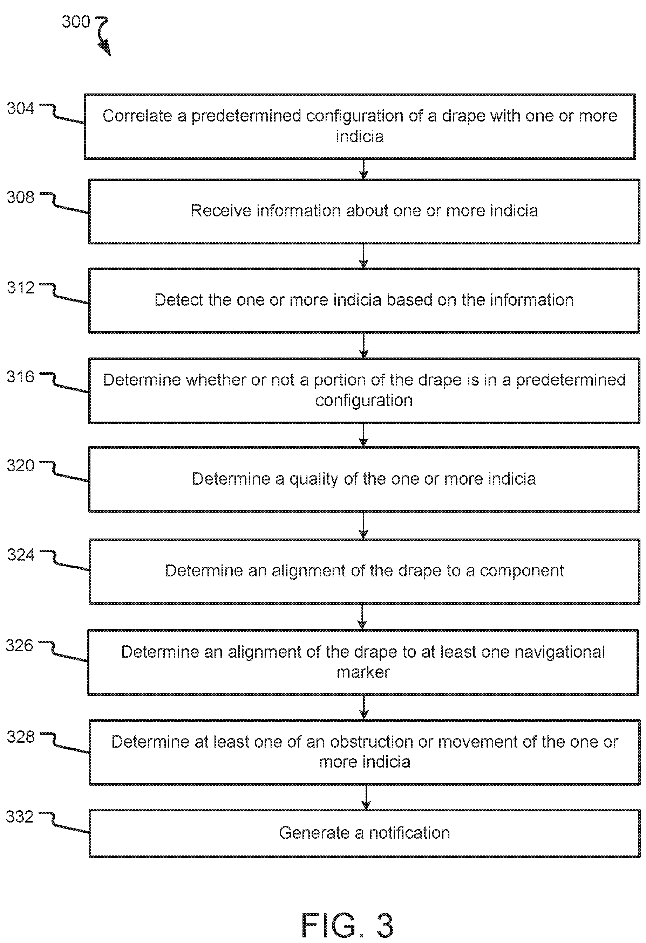

During use, as will be described in detail with respect to Figs. 3 and 4, the sensor 212 may sense the at least one indicia 106 and transmit information (such as, for example, imaging information and/or processed image data) about the at least one indicia 106 to the computing device 202 or a processor 204 (shown in Fig. 2). The processor 204 may determine, based on the information, whether the drape 102 or the portion 108 of the drape 102 is in a predetermined configuration. In some embodiments, the predetermined configuration may correlate to whether the drape 102 or the portion 108 of the drape 102 is sufficiently flat or positioned so as to provide a clear view of the component 104 underneath the portion 108 of the drape 102. For example, if the portion 108 is wrinkled, then a shape of the at least one indicia 106 may be deformed (such as, for example, when the at least one indicia 106 is a straight light, the straight line may be crooked), then a view of the component 104 or navigation markers 103 may not be clear. Such wrinkle, distortion, or deformation of the at least one indicia 106 may prevent, for example, a navigation camera from detecting a marker positioned underneath the portion 108. In other embodiments, the predetermined configuration of the drape 102 and/or the predetermined configuration may correlate to whether the drape 102 is in a configuration that allows for movement of, for example, a robotic arm such as the robotic arm 216. For example, if the drape 102 is not in the predetermined configuration, the drape 102 may have a higher likelihood of becoming snagged or caught on the robot arm 216.

In some embodiments, the information may be image information (e.g., image data, pixel data, etc.) received and analyzed by the processor 204 to determine a configuration of the at least one indicia 106. The at least one indicia 106 may be correlated to a configuration of the drape 102. The measured configuration of the drape 102, as analyzed by the processor 204, can be compared to a predetermined or desired configuration of the drape 102 to determine if the drape 102 is properly installed and/or has retained an appropriate configuration.

In other embodiments, the at least one indicia 106 may be used to verify or confirm that the drape 102 is compatible with the system 100, 200 being used for a surgical procedure. In such embodiments, the system 100, 200 may not allow for use of some or all components of the system 100, 200 until the at least one indicia 106 is confirmed.

Also shown in the illustrated embodiment, the at least one indicia 106 may include a first set of indicia 106A disposed on a first portion 108A of the drape 102 and a second set of indicia 106B disposed on a second portion 108B of the drape 102. In such embodiments, the first set of indicia 106A and the second set of indicia 106B may be used to determine whether the drape 102 at the first portion 108A and/or the second portion 108B is in a predetermined configuration. In other embodiments, a first set of indicia may be disposed on the portion 108 the drape 102 and a second set of indicia may be disposed on the portion 110 of the component 104. In such embodiments, the first set of indicia and the second set of indicia may be used to align the portion 108 of the drape 102 with the portion 110 of the component 104. It will be appreciated that the at least one indicia 106 may include any number of sets of indicia. Further, each set of indicia may be the same as or similar to each other or each set may be different from each other.

Turning to Fig. 1B, a component 104' and a portion 108' of a drape 102' is shown separate from the component 104'. The component 104' is the same as or similar to the component 104 described above. The drape 102' is the same as or similar to the drape 102 described above. As shown in the illustrated embodiment, at least one indicia 106', which may be the same as or similar to the at least one indicia 106, is disposed, affixed, or otherwise integrated with a portion 110 of the component 104'. Also shown in the illustrated embodiment, a first set of indicia 106A' - which may be the same as or similar to the first set of indicia 106A - may be disposed on a first portion 110A of the component 104' and a second set of indicia 106B' - which may be the same as or similar to the second set of indicia 106B - may be disposed on a second portion 110B of the component 104'. The first set of indicia 106A' is disposed near at least one navigational marker 103' which may be the same as or similar to the at least one navigational marker 103. It will be appreciated that any number of sets of indicia may be disposed on or integrated with the component 104'.

During use, which will be further described below with respect to Figs. 3 and 4 and similarly described above, the at least one indicia 106' may be used to determine if the drape 102' is in a predetermined configuration. In some embodiments, the sensor 212 may sense the at least one indicia 106' and transmit information about the at least one indicia 106' to the computing device 202 or the processor 204 (shown in Fig. 2). The processor 204 may determine, based on the information, whether the drape 102 or the portion 108 of the drape 102 is in a predetermined configuration.

Also shown in the illustrated embodiment, a third set of indicia 106C may be disposed on the portion 108' of the drape 102'. The third set of indicia 106C may match the second set of indicia 106B' of the component 104'. In such embodiments, the drape 102' may be aligned to the component 104' by aligning the third set of indicia 106C to the second set of indicia 106B'. Such alignment may, for example, aid in positioning the drape 102' in a predetermined configuration. In other embodiments, the third set of indicia 106C may be aligned to a portion of the component 104' that does not have a set of indicia. For example, the third set of indicia 106C may be positioned or aligned to a joint of a robotic arm.

Turning to Fig. 2, a block diagram of a system 200 according to at least one embodiment of the present disclosure is shown. The system 200 may be used to detect and/or monitor a drape configuration and/or carry out one or more other aspects of one or more of the methods disclosed herein. The system 200 comprises a computing device 202, one or more sensors 212, a robot 214, a navigation system 218, a database 230, and/or a cloud or other network 234. Systems according to other embodiments of the present disclosure may comprise more or fewer components than the system 200. For example, the system 200 may not include the sensor 212, the robot 214, the navigation system 218, one or more components of the computing device 202, the database 230, and/or the cloud 234.

The computing device 202 comprises a processor 204, a memory 206, a communication interface 208, and a user interface 210. Computing devices according to other embodiments of the present disclosure may comprise more or fewer components than the computing device 202.

The processor 204 of the computing device 202 may be any processor described herein or any similar processor. The processor 204 may be configured to execute instructions stored in the memory 206, which instructions may cause the processor 204 to carry out one or more computing steps utilizing or based on data received from the sensor 212, the robot 214, the navigation system 218, the database 230, and/or the cloud 234.

The memory 206 may be or comprise RAM, DRAM, SDRAM, other solid-state memory, any memory described herein, or any other tangible, non-transitory memory for storing computer-readable data and/or instructions. The memory 206 may store information or data useful for completing, for example, any step of the method 300 described herein, or of any other methods. The memory 206 may store, for example, one or more algorithms 220 and/or one or more image processing algorithms 222. Such instructions or algorithms may, in some embodiments, be organized into one or more applications, modules, packages, layers, or engines. Alternatively or additionally, the memory 206 may store other types of data (e.g., machine learning modes, artificial neural networks, etc.) that can be processed by the processor 204 to carry out the various method and features described herein. Thus, although various components of memory 206 are described as instructions, it should be appreciated that functionality described herein can be achieved through use of instructions, algorithms, and/or machine learning models. The data, algorithms, and/or instructions may cause the processor 204 to manipulate data stored in the memory 206 and/or received from or via the sensor 212, the robot 214, the database 230, and/or the cloud 234.

The computing device 202 may also comprise a communication interface 208. The communication interface 208 may be used for receiving sensor data or other information from an external source (such as the sensor 212, the robot 214, the navigation system 218, the database 230, the cloud 234, and/or any other system or component not part of the system 200), and/or for transmitting instructions, images, or other information to an external system or device (e.g., another computing device 202, the sensor 212, the robot 214, the navigation system 218, the database 230, the cloud 234, and/or any other system or component not part of the system 200). The communication interface 208 may comprise one or more wired interfaces (e.g., a USB port, an ethernet port, a Firewire port) and/or one or more wireless transceivers or interfaces (configured, for example, to transmit and/or receive information via one or more wireless communication protocols such as 802.11a/b/g/n, Bluetooth, NFC, ZigBee, and so forth). In some embodiments, the communication interface 208 may be useful for enabling the device 202 to communicate with one or more other processors 204 or computing devices 202, whether to reduce the time needed to accomplish a computing-intensive task or for any other reason.

The computing device 202 may also comprise one or more user interfaces 210. The user interface 210 may be or comprise a keyboard, mouse, trackball, monitor, television, screen, touchscreen, and/or any other device for receiving information from a user and/or for providing information to a user. The user interface 210 may be used, for example, to receive a user selection or other user input regarding any step of any method described herein. Notwithstanding the foregoing, any required input for any step of any method described herein may be generated automatically by the system 200 (e.g., by the processor 204 or another component of the system 200) or received by the system 200 from a source external to the system 200. In some embodiments, the user interface 210 may be useful to allow a surgeon or other user to modify instructions to be executed by the processor 204 according to one or more embodiments of the present disclosure, and/or to modify or adjust a setting of other information displayed on the user interface 210 or corresponding thereto.

Although the user interface 210 is shown as part of the computing device 202, in some embodiments, the computing device 202 may utilize a user interface 210 that is housed separately from one or more remaining components of the computing device 202. In some embodiments, the user interface 210 may be located proximate one or more other components of the computing device 202, while in other embodiments, the user interface 210 may be located remotely from one or more other components of the computer device 202.

The system 200 includes the one or more sensor(s) 212. The sensor 212 is operable to detect the at least one indicia 106, 106' (described in detail with respect to Figs. 1A-1B) and yield sensor data. The sensor data may be or comprise preoperative sensor data, intraoperative sensor data, postoperative sensor data, or sensor data obtained independently of any surgical procedure. The sensor 212 may be, for example, a high-definition camera, an optical camera; an infrared camera; a 3D camera system; a stereoscopic vision system; an imaging device; or any other sensor that can detect the at least one indicia 106, 106'. In some examples, the at least one indicia 106, 106' may be active (e.g., an infrared light emitting diode) and the sensor 212 may be configured to sense the at least one indicia 106, 106' when the at least one indicia 106, 106' are activated. In other examples, the at least one indicia 106, 106' may be passive (e.g., reflective markers).

The sensor 212 may comprise a dedicated processor for executing instructions stored in a dedicated memory of the sensor 212, or the sensor 212 may simply be configured to transmit data collected therewith to the computing device 202 or to another component of the system 200. Although shown in Fig. 2 as being in communication only with the computing device 202, in some embodiments, the sensor 212 may be in communication with any one or more of the robot 214, the navigation system 218, the database 230, and/or the cloud 234. Also, in some embodiments, the computing device 202 may comprise the sensor 212, while in other embodiments, the navigation system 218 may comprise the sensor 212. In still other embodiments, the robot 214 may comprise the sensor 212.

The sensor 212 may be configured to capture data regarding sensed indicia 106, 106' only at a given moment in time. For example, where the sensor 212 is a camera, the sensor 212 may be configured to capture still images comprising the at least one indicia 106, 106'. The sensor 212 may be configured to capture such data at periodic intervals, or when commanded by a user (e.g., via a user interface 210), or upon a signal (generated either autonomously or in response to user input) from the computing device 202, the robot 214, and/or the navigation system 218. In some embodiments, a first sensor may be used to obtain first sensor data at a first time, and a second sensor may be used to obtain second sensor data at a second time after the first time.

The sensor 212 may additionally or alternatively be operable to capture data corresponding to the at least one indicia 106, 106' continuously, in real-time. In such embodiments, the sensor 212 may provide a stream of real-time sensor data to the computing device 202, which may continuously process the sensor data to detect the at least one indicia 106, 106'.

In some embodiments, the sensor 212 may be an imaging device operable to image anatomical feature(s) (e.g., a bone, veins, tissue, etc.) and/or other aspects of patient anatomy to yield image data (e.g., image data depicting or corresponding to a bone, veins, tissue, etc.). "Image data" as used herein refers to the data generated or captured by an imaging device, including in a machine-readable form, a graphical/visual form, and in any other form. In various examples, the image data may comprise data corresponding to an anatomical feature of a patient, or to a portion thereof. The imaging device may be capable of taking a 2D image or a 3D image to yield the image data. The imaging device may be or comprise, for example, an ultrasound scanner (which may comprise, for example, a physically separate transducer and receiver, or a single ultrasound transceiver), an O-arm, a C-arm, a G-arm, or any other device utilizing X-ray-based imaging (e.g., a fluoroscope, a CT scanner, or other X-ray machine), a magnetic resonance imaging (MRI) scanner, an optical coherence tomography (OCT) scanner, an endoscope, a microscope, an optical camera, a thermographic camera (e.g., an infrared camera), a radar system (which may comprise, for example, a transmitter, a receiver, a processor, and one or more antennae), or any other imaging device suitable for obtaining images of an anatomical feature of a patient. The imaging device may be contained entirely within a single housing, or may comprise a transmitter/emitter and a receiver/detector that are in separate housings or are otherwise physically separated.

The system 200 may also include the robot 214. The robot 214 may be any surgical robot or surgical robotic system. The robot 214 may be or comprise, for example, the Mazor X^{™} Stealth Edition robotic guidance system. The robot 214 may be configured to position the sensor 212 at one or more precise position(s) and orientation(s), and/or to return the sensor 212 to the same position(s) and orientation(s) at a later point in time. The robot 214 may additionally or alternatively be configured to manipulate a surgical tool (whether based on guidance from the navigation system 218 or not) to accomplish or to assist with a surgical task. In some embodiments, the robot 214 may be configured to hold and/or manipulate an anatomical element during or in connection with a surgical procedure. The robot 214 may comprise one or more robotic arms 216. In some embodiments, the robotic arm 216 may comprise a first robotic arm and a second robotic arm, though the robot 214 may comprise more than two robotic arms. In some embodiments, one or more of the robotic arms 216 may be used to hold and/or maneuver the sensor 212. In embodiments where the sensor 212 comprises two or more physically separate components (e.g., a transmitter and receiver), one robotic arm 216 may hold one such component, and another robotic arm 216 may hold another such component. Each robotic arm 216 may be positionable independently of the other robotic arm. The robotic arms may be controlled in a single, shared coordinate space, or in separate coordinate spaces.

The robot 214, together with the robotic arm 216, may have, for example, one, two, three, four, five, six, seven, or more degrees of freedom. Further, the robotic arm 216 may be positioned or positionable in any pose, plane, and/or focal point. The pose includes a position and an orientation. As a result, a sensor 212, surgical tool, or other object held by the robot 214 (or, more specifically, by the robotic arm 216) may be precisely positionable in one or more needed and specific positions and orientations.

The robotic arm(s) 216 may comprise one or more sensors that enable the processor 204 (or a processor of the robot 214) to determine a precise pose in space of the robotic arm (as well as any object or element held by or secured to the robotic arm 216).

In some embodiments, reference markers (i.e., navigation markers) may be placed on the robot 214 (including, e.g., on the robotic arm 216), the sensor 212, the component 104, or any other object in the surgical space. The reference markers may be tracked by the navigation system 218, and the results of the tracking may be used by the robot 214 and/or by an operator of the system 200 or any component thereof. In some embodiments, the navigation system 218 can be used to track other components of the system (e.g., sensor 212) and the system can operate without the use of the robot 214 (e.g., with the surgeon manually manipulating the sensor 212 and/or one or more surgical tools, based on information and/or instructions generated by the navigation system 218, for example).

The system 200 may also include the navigation system 218. The navigation system 218 may provide navigation for a surgeon and/or a surgical robot during an operation. The navigation system 218 may be any now-known or future-developed navigation system, including, for example, the Medtronic StealthStation^{™} S8 surgical navigation system or any successor thereof. The navigation system 218 may include one or more cameras or other sensor(s) for tracking one or more reference markers, navigated trackers, or other objects within the operating room or other room in which some or all of the system 200 is located. The one or more cameras may be optical cameras, infrared cameras, or other cameras. In some embodiments, the navigation system may comprise one or more electromagnetic sensors. In various embodiments, the navigation system 218 may be used to track a position and orientation (i.e., pose) of the component 104, the sensor 212, the robot 214 and/or robotic arm 216, and/or one or more surgical tools (or, more particularly, to track a pose of a navigated tracker attached, directly or indirectly, in fixed relation to the one or more of the foregoing). The navigation system 218 may include a display for displaying one or more images from an external source (e.g., the computing device 202, sensor 212, or other source) or for displaying an image and/or video stream from the one or more cameras or other sensors of the navigation system 218. In some embodiments, the system 200 can operate without the use of the navigation system 218. The navigation system 218 may be configured to provide guidance to a surgeon or other user of the system 200 or a component thereof, to the robot 214, or to any other element of the system 200 regarding, for example, a pose of one or more anatomical elements, whether or not a tool is in the proper trajectory, and/or how to move a tool into the proper trajectory to carry out a surgical task according to a preoperative or other surgical plan.

The system 200 or similar systems may be used, for example, to carry out one or more aspects of any of the method 300 described herein. The system 200 or similar systems may also be used for other purposes.

Fig. 3 depicts a method 300 that may be used, for example, for detecting and/or monitoring a configuration of a drape.

The method 300 (and/or one or more steps thereof) may be carried out or otherwise performed, for example, by at least one processor. The at least one processor may be the same as or similar to the processor(s) 204 of the computing device 202 described above. The at least one processor may be part of a robot (such as a robot 214) or part of a navigation system (such as a navigation system 218). A processor other than any processor described herein may also be used to execute the method 300. The at least one processor may perform the method 300 by executing instructions stored in a memory such as the memory 206. The instructions may correspond to one or more steps of the method 300 described below. The instructions may cause the processor to execute one or more algorithms, such as an algorithm 220 and/or an image processing algorithm 222.

The method 300 comprises correlating a predetermined configuration of a drape with one or more indicia (step 304). The one or more indicia, which may be the same as or similar to the one or more indicia 106, 106', may be disposed on or otherwise integrated with a portion, such as the portion 108, 108', of a drape, such as the drape 102, 102', or a portion, such as the portion 110, of a component 104, 104'.

Correlating the predetermined configuration of the drape with the one or more indicia may include receiving information about a layout, arrangement, or pattern of the one or more indicia when the drape is in the predetermined configuration by a processor such as the processor 204. For example, the information may include sensor data (received from, for example, a sensor such as the sensor 212) or image data (received from, for example, an imaging device) of the one or more indicia when the drape is in the predetermined configuration. The information may be received from any component of a system such as the system 100, 200, from a user interface such as the user interface 210, or a source external to the system. The received information may be used by the processor to correlate the one or more indicia to the predetermined configuration. For example, in some embodiments, the correlation may include that the one or more indicia is visible (whether to, for example, the sensor, the imaging device, or a user) when the portion of the drape is in a predetermined configuration.

The method 300 comprises receiving information about the one or more indicia (step 308). In some embodiments, the information may be received by, for example, the sensor, the imaging device, or the user via a user interface such as the user interface 210. In other embodiments, the information may be received from any component of the system 100, 200 or from a source external to the system 100, 200.

The information about the one or more indicia may comprise, in some embodiments, sensor data received from the sensor. For example, the one or more indicia may comprise infrared light emitting diodes and the sensor may be an infrared sensor configured to detect the infrared light emitting diodes and yield sensor data. In other embodiments, the information about the one or more indicia may comprise image data obtained from, for example, an imaging device. The image data may depict the one or more indicia. In still other embodiments, the information may comprise pose (e.g., a position and an orientation) data about the one or more indicia.

The method 300 also comprises detecting the one or more indicia based on the information (step 312). The information may be received from, for example, step 308. In embodiments where the information comprises image data, detecting the one or more indicia may comprise processing the image data by a processor such as the processor 204 using an image processing algorithm such as the image processing algorithm 222. Such image processing algorithm may, for example, segment the image data to detect the one or more indicia. In other embodiments, the sensor data may be processed by the processor to detect the one or more indicia.

In embodiments where the one or more indicia comprises a first set of indicia and a second set of indicia, step 312 may comprise detecting the first set of one or more indicia and the second set of one or more indicia. In such embodiments, the first set of indicia and the second set of indicia may be disposed on or otherwise integrated with the drape. In other embodiments, the first set of indicia and the second set of indicia may be disposed on or otherwise integrated with the component. In still other embodiments, the first set of indicia may be disposed on or otherwise integrated with the drape and the second set of indicia may be disposed on or otherwise integrated with the component.

The method 300 also comprises determining whether or not the portion of the drape is in a predetermined configuration (step 316). The predetermined configuration or shape may correlate to the portion of the drape being substantially flat. In other embodiments, the predetermined configuration may correlate to the portion of the drape being configured so as to provide an unobstructed line of sight to and from the portion of the component underneath the drape. For example, it may be desirable to maintain an unobstructed line of sight to a marker underneath the drape so that a navigation system such as the navigation system 218 may be able to detect the marker. In other example, it may be desirable to maintain an unobstructed line of sight to a camera so that the camera may operate without any obstructions. In still other embodiments, the predetermined configuration may correlate to the portion of the drape being configured so as to allow for movement of the component. For example, the drape may be draped over a robotic arm and configured to allow for movement of the robotic arm. In other embodiments, the predetermined configuration may correlate to a layout or arrangement of the one or more indicia as detected by the sensor. The layout or arrangement of the one or more indicia may correlate to the drape being in the predetermined configuration.

The step 316 may also comprise determining if the one or more indicia are visible to the, for example, the sensor or the imaging device and if the visible one or more indicia match a predetermined indicia. The predetermined indicia may be the same as the information about one or more indicia received and correlated in step 304. In other embodiments, the predetermined indicia may be received as input from the user interface or received from any other component or source. In some embodiments, if the one or more indicia are visible to the sensor or the imaging device and match the predetermined indicia, this may indicate that the portion of the drape is positioned in the predetermined configuration. If the one or more indicia are not visible to the sensor or the imaging device and/or do not match the predetermined indicia, this may indicate that the portion of the drape is not positioned in the predetermined configuration. For example, the one or more indicia may be visible to the sensor or the imaging device, but may not match a predetermined indicia. For example, the detected one or more indicia may be in the shape of a crescent, however, the predetermined indicia may be in the shape of a circle. This may indicate that the drape is wrinkled or otherwise not in the predetermined configuration. In another example, the predetermined indicia may be straight lines, however, the detected one or more indicia may be zig-zag lines, which may indicate that placement of the drape was inaccurate or that the drape is otherwise not in the predetermined configuration.

The step 316 may also comprise comparing the detected one or more indicia to a predetermined indicia (whether the one or more indicia are determined to be visible to a sensor or an imaging device) as described above.

The method 300 also comprises determining a quality of the one or more indicia (step 320). In some embodiments, the quality of the one or more indicia may correlate to a degree of flatness of the portion of the drape. In other embodiments, the quality may correlate to a proportion of the portion of the drape that is in the predetermined configuration.

Determining the quality of the one or more indicia may include matching the one or more indicia to a predetermined indicia and determining a percentage or proportion of the one or more indicia that matches the predetermined indicia.

The method 300 also comprises determining an alignment of the drape to the component (step 324). In embodiments where the one or more indicia comprises a first set of indicia disposed on or otherwise integrated with the drape and a second set of indicia is disposed on or otherwise integrated with the component, determining an alignment of the drape to the component may be based on an alignment of the first set of indicia to the second set of indicia. In other embodiments where the one or more indicia may be disposed on or otherwise integrated with the drape, the one or more indicia may be aligned to a portion of the component that does not have a set of indicia. For example, the one or more indicia may be positioned or aligned to a joint of a robotic arm.

The method 300 also comprises aligning the at least one indicia and at least one navigational marker (step 326). The at least one navigational marker may be the same as or similar to the at least one navigational marker 103. In some embodiments, the at least one navigational marker may be disposed on or affixed to the component. In embodiments where the at least one indicia is integrated with the drape, the at least one indicia may be positioned near or aligned with the at least one navigational marker when the drape is positioned over the component. In embodiments where the at least one indicia is disposed on or otherwise integrated with the component, the at least one navigational marker may be positioned near or aligned with the at least one indicia.

The method 300 also comprises determining at least one of an obstruction or movement of the one or more indicia (step 328). Determining the at least one of an obstruction or movement of the one or more indicia may be based on the information about the one or more indicia received in step 308 and/or whether the one or more indicia are detected in step 312. For example, if the one or more indicia are not detected in step 312, this may indicate that the one or more indicia are not visible to the sensor or the camera or may be distorted, whether due to an obstruction, movement of the drape, or otherwise. In examples where the information includes pose information, pose information at a first time may be compared to pose information at a second time to determine whether the one or more indicia has moved.

The method 300 also comprises generating a notification (step 332). The notification may be a visual notification, an audible notification, or any type of notification communicated to a user. The notification may be communicated to the user via a user interface such as the user interface 210. In some embodiments, the notification may be automatically generated by the processor 204. In other embodiments, the notification may be automatically generated by any component of a system such as the system 200.

The notification may be generated when: the drape is not in the predetermined configuration, as determined in, for example, step 316; when the drape is not aligned to the component as determined in, for example, step 324; when at least one of an obstruction or movement of the one or more indicia is detected in, for example, step 328; and/or for any other reason. In some embodiments, the notification may include the quality of the one or more indicia determined, for example, in step 316. In other embodiments, the notification may include the degree of flatness of the portion of the drape and/or the proportion of the portion of the drape that is in the predetermined configuration.

In some embodiments, the notification may be based on a predetermined threshold difference between the detected one or more indicia and a predetermined indicia. The threshold difference may correlate to a maximum allowable difference for the difference and the notification may be generated when the difference meets or exceeds the corresponding threshold difference. For example, the threshold difference may allow for a one percent different between the detected one or more indicia and the predetermined indicia. The threshold difference may be determined automatically using artificial intelligence and training data (e.g., historical cases) in some embodiments. In other embodiments, the threshold difference may be or comprise, or be based on, surgeon input received via the user interface. In further embodiments, the threshold difference may be determined automatically using artificial intelligence, and may thereafter be reviewed and approved (or modified) by a surgeon or other user. In examples where the one or more indicia includes two or more sets of indicia, a notification may be generated for each set of indicia that meets or exceeds the corresponding threshold difference. The notification may alert a surgeon or user of a difference between the detected indicia and the predetermined indicia that the surgeon or other user may wish to review or otherwise mitigate.

It will be appreciated that the steps 302-332 can occur at any time. For example, the steps 308-332 may be continuously repeated throughout a surgical procedure to monitor and confirm that the drape is in the predetermined configuration. In other examples, the steps 308-332 may occur upon input from, for example, a user such as a surgeon or other medical provider. In still other examples, the steps 308-332 may occur at a time interval throughout a surgical procedure. In other examples, the step 304 may be repeated if, for example, the drape is moved by a user such as a surgeon or other medical provider. In another example, the step 304 may be repeated if the drape is moved to a new position by the component (for example, when the component is a robotic arm such as the robotic arm 216) and it is desirable to monitor the drape in the new position.

It will also be appreciated that the steps 302-332 may be used to verify that the drape is correctly positioned. In some cases, components of a system such as the system 100, 200 may be locked or otherwise unusable until verification of the drape positioning occurs.

The present disclosure encompasses embodiments of the method 300 that comprise more or fewer steps than those described above, and/or one or more steps that are different than the steps described above.

As noted above, the present disclosure encompasses methods with fewer than all of the steps identified in Fig. 3 (and the corresponding description of the method 300), as well as methods that include additional steps beyond those identified in Fig. 3 (and the corresponding description of the method 300). The present disclosure also encompasses methods that comprise one or more steps from one method described herein, and one or more steps from another method described herein. Any correlation described herein may be or comprise a registration or any other correlation.

The foregoing is not intended to limit the disclosure to the form or forms disclosed herein. In the foregoing Detailed Description, for example, various features of the disclosure are grouped together in one or more aspects, embodiments, and/or configurations for the purpose of streamlining the disclosure.

## Claims

1. A system (100, 200), comprising:
a surgical drape (102);
one or more indicia (106), wherein the one or more indicia (106) is visible when at least a portion of the drape (102) is in a predetermined configuration, wherein the predetermined configuration is when the portion of the drape is substantially flat;
one or more sensors (212) configured for sensing the at least one indicia (106) and transmitting information about the at least one indicia (106) to a processor (204);
the at least one processor (204); and
a memory (206) storing data for processing by the at least one processor (204) that, when processed by the at least one processor (204), causes the at least one processor (204) to:
receive the information about the one or more indicia (106),
determine, based on the information, whether or not the portion of the drape (102) is in the predetermined configuration by determining a quality of the one or more indicia (106) based on the information, the quality of the one or more indicia (106) correlating to a degree of flatness of the portion of the drape (102), and
generate a notification when the portion of the drape (102) is not in the predetermined configuration, the notification including the degree of flatness of the portion of the drape (102).

2. The system (100, 200) of claim 1, wherein determining whether or not the portion of the drape (102) is in the predetermined configuration further comprises comparing the detected one or more indicia (106) to a predetermined indicia, wherein the portion of the drape (102) is not in the predetermined configuration when the one or more indicia (106) does not substantially match the predetermined indicia.

3. The system (100, 200) of claim 1, wherein the one or more indicia (106) is at least one of a QR code, magnetic dots, a barcode, or a pattern.

4. The system (100, 200) of claim 1, wherein the one or more indicia (106) is positioned near at least one navigational marker (103) affixed to a surgical device (104, 214) when the drape (102) is positioned on the surgical device (104, 214).

5. The system (100, 200) of claim 1, wherein the sensor (212) is a camera and the information is an image received from the camera.

6. The system (100, 200) of claim 1, wherein the one or more indicia (106) is a first set of one or more indicia (106A) and wherein the system (100, 200) further comprises a second set of one or more indicia (106B) disposed on a component.

7. The system (100, 200) of claim 6, wherein detecting the one or more indicia (106) comprises detecting the first set of one or more indicia (106A) and the second set of one or more indicia (106B), wherein the memory (206) stores additional data for execution by the at least one processor (204) that, when executed, further cause the at least one processor (204) to:
determine an alignment of the drape (102) to the surgical device (104, 214) based on an alignment of the first set of one or more indicia (106A) to the second set of one or more indicia (106B).

8. The system (100, 200) of claim 1, wherein the memory (206) stores additional data for execution by the at least one processor (204) that, when executed, further cause the at least one processor (204) to:
receive an image from a camera, and
determine, from the image, at least one of an obstruction of the one or more indicia (106) or movement of the one or more indicia (106).

9. The system (100, 200) of claim 1, wherein the one or more indicia (106) comprises at least one optically visible indicia and at least one infrared marker.

10. The system (100, 200) of claim 1, wherein the one or more indicia (106) is disposed on at least a portion of the drape (102).

11. The system (100, 200) of claim 1, wherein the information is at least one image obtained from the sensor (212) and detecting the one or more indicia (106) includes processing the at least one image using an image processing algorithm to detect the one or more indicia (106).

12. The system (100, 200) of claim 1, wherein the one or more indicia (106) is disposed on the drape (102).

13. The system (100, 200) of claim 1, wherein the one or more indicia (106) is disposed on a component positioned underneath the drape (102).

## Patentansprüche

1. System (100, 200), umfassend:
ein Operationsabdecktuch (102);
ein oder mehrere Zeichen (106), wobei das eine oder die mehreren Zeichen (106) sichtbar ist, wenn mindestens ein Abschnitt des Abdecktuchs (102) in einer zuvor bestimmten Konfiguration ist, wobei die zuvor bestimmte Konfiguration vorliegt, wenn der Abschnitt des Abdecktuchs im Wesentlichen flach ist;
einen oder mehrere Sensoren (212), die zum Erfassen des mindestens einen Zeichens (106) und zum Übertragen von Informationen über das mindestens eine Zeichen (106) an einen Prozessor (204) konfiguriert sind;
den mindestens einen Prozessor (204); und
einen Speicher (206), der Daten zum Verarbeiten durch den mindestens einen Prozessor (204) speichert, die, wenn sie durch den mindestens einen Prozessor (204) verarbeitet sind, den Prozessor (204) veranlassen zum:
Empfangen der Informationen über das eine oder die mehreren Zeichen (106),
Bestimmen, basierend auf den Informationen, ob der Abschnitt des Abdecktuchs (102) in der zuvor bestimmten Konfiguration ist oder nicht, durch Bestimmen einer Qualität des einen oder der mehreren Zeichen (106) basierend auf den Informationen, wobei die Qualität des einen oder der mehreren Zeichen (106) mit einem Grad von Flachheit des Abschnitts des Abdecktuchs (102) korreliert, und
Erzeugen einer Benachrichtigung, wenn der Abschnitt des Abdecktuchs (102) nicht in der zuvor bestimmten Konfiguration ist, die Benachrichtigung einschließlich eines Grads von Flachheit des Abschnitts des Abdecktuchs (102).

2. System (100, 200) nach Anspruch 1, wobei das Bestimmen, ob der Abschnitt des Abdecktuchs (102) in der zuvor bestimmten Konfiguration ist oder nicht, ferner ein Vergleichen des detektierten einen oder der mehreren Zeichen (106) mit einem zuvor bestimmten Zeichen umfasst, wobei der Abschnitt des Abdecktuchs (102) nicht in der zuvor bestimmten Konfiguration ist, wenn das eine oder die mehreren Zeichen (106) im Wesentlichen nicht mit dem zuvor bestimmten Zeichen übereinstimmt.

3. System (100, 200) nach Anspruch 1, wobei das eine oder die mehreren Zeichen (106) mindestens eines ist von einem QR-Code, magnetischen Punkten, einem Strichcode oder einem Muster.

4. System (100, 200) nach Anspruch 1, wobei das eine oder die mehreren Zeichen (106) nahe mindestens einer Navigationsmarkierung (103) positioniert ist, die an einer Operationsvorrichtung (104, 214) befestigt ist, wenn das Abdecktuch (102) auf der Operationsvorrichtung (104, 214) positioniert ist.

5. System (100, 200) nach Anspruch 1, wobei der Sensor (212) eine Kamera ist und die Information ein Bild sind, das von der Kamera empfangen wird.

6. System (100, 200) nach Anspruch 1, wobei das eine oder die mehreren Zeichen (106) ein erster Satz von einem oder mehreren Zeichen (106A) ist, und wobei das System (100, 200) ferner einen zweiten Satz von einem oder mehreren Zeichen (106B), die an einer Komponente angeordnet sind, umfasst.

7. System (100, 200) nach Anspruch 6, wobei das Detektieren des einen oder der mehreren Zeichen (106) das Detektieren des ersten Satzes von einem oder mehreren Zeichen (106A) und des zweiten Satzes von einem oder mehreren Zeichen (106B) umfasst, wobei der Speicher (206) zusätzliche Daten für eine Ausführung durch den mindestens einen Prozessor (204) speichert, die, wenn sie ausgeführt sind, den mindestens einen Prozessor (204) ferner veranlassen zum:
Bestimmen einer Ausrichtung des Abdecktuchs (102) zu der Operationsvorrichtung (104, 214) basierend auf einer Ausrichtung des ersten Satzes von einem oder mehreren Zeichen (106A) zu dem zweiten Satz von einem oder mehreren Zeichen (106B).

8. System (100, 200) nach Anspruch 1, wobei der Speicher (206) zusätzliche Daten für die Ausführung durch den mindestens einen Prozessor (204) speichert, die, wenn sie ausgeführt sind, den mindestens einen Prozessor (204) ferner veranlassen zum:
Empfangen eines Bildes von einer Kamera, und
Bestimmen, aus dem Bild, mindestens einer von einer Verdeckung des einen oder der mehreren Zeichen (106) oder einer Bewegung des einen oder der mehreren Zeichen (106).

9. System (100, 200) nach Anspruch 1, wobei das eine oder die mehreren Zeichen (106) mindestens ein optisch sichtbares Zeichen und mindestens eine Infrarotmarkierung umfasst.

10. System (100, 200) nach Anspruch 1, wobei das eine oder die mehreren Zeichen (106) auf mindestens einem Abschnitt des Abdecktuchs (102) angeordnet ist.

11. System (100, 200) nach Anspruch 1, wobei die Information mindestens ein Bild sind, das von dem Sensor (212) erhalten wird, und das Detektieren des einen oder der mehreren Zeichen (106) das Verarbeiten des mindestens einen Bildes unter Verwendung eines Bildverarbeitungsalgorithmus, um das eine oder die mehreren Zeichen (106) zu detektieren, einschließt.

12. System (100, 200) nach Anspruch 1, wobei das eine oder die mehreren Zeichen (106) auf dem Abdecktuch (102) angeordnet ist.

13. System (100, 200) nach Anspruch 1, wobei das eine oder die mehreren Zeichen (106) auf einer Komponente, die unter dem Abdecktuch (102) positioniert ist, angeordnet ist.

## Revendications

1. Système (100, 200), comprenant :
un champ opératoire (102) ;
une ou plusieurs indications (106), dans lequel l'une ou les indications (106) sont visibles lorsqu'au moins une partie du champ (102) est dans une configuration prédéterminée, dans lequel la configuration prédéterminée est lorsque la partie du champ est sensiblement plate ;
un ou plusieurs capteurs (212) configurés pour détecter l'au moins une indication (106) et transmettre des informations sur l'au moins une indication (106) à un processeur (204) ;
l'au moins un processeur (204) ; et
une mémoire (206) stockant des données destinées à être traitées par l'au moins un processeur (204) qui, lorsqu'elles sont traitées par l'au moins un processeur (204), amènent l'au moins un processeur (204) à :
recevoir les informations relatives à l'indication ou aux indications (106),
déterminer, en fonction des informations, si la partie du champ (102) est ou non dans la configuration prédéterminée en déterminant une qualité de la ou des indications (106) en fonction des informations, la qualité de l'une ou des indications (106) étant corrélée à un degré de planéité de la partie du champ (102), et
générer une notification lorsque la partie du champ (102) n'est pas dans la configuration prédéterminée, la notification comportant le degré de planéité de la partie du champ (102).

2. Système (100, 200) selon la revendication 1, dans lequel le fait de déterminer si la partie du champ (102) est ou non dans la configuration prédéterminée comprend en outre la comparaison de l'une ou des indications détectées (106) à une indication prédéterminée, dans lequel la partie du champ (102) n'est pas dans la configuration prédéterminée lorsque l'une ou les indications (106) ne correspondent pas sensiblement à l'indication prédéterminée.

3. Système (100, 200) selon la revendication 1, dans lequel l'une ou les indications (106) sont au moins l'une parmi un QR code, des points magnétiques, un code-barres ou un motif.

4. Système (100, 200) selon la revendication 1, dans lequel l'une ou les indications (106) sont positionnées à proximité d'au moins un marqueur de navigation (103) fixé à un dispositif chirurgical (104, 214) lorsque le champ opératoire (102) est positionné sur le dispositif chirurgical (104, 214).

5. Système (100, 200) selon la revendication 1, dans lequel le capteur (212) est une caméra et les informations sont une image reçue de la caméra.

6. Système (100, 200) selon la revendication 1, dans lequel l'une ou les indications (106) sont un premier ensemble d'une ou plusieurs indications (106A) et dans lequel le système (100, 200) comprend en outre un second ensemble d'une ou plusieurs indications (106B) disposées sur un composant.

7. Système (100, 200) selon la revendication 6, dans lequel la détection de l'une ou plusieurs indications (106) comprend la détection du premier ensemble d'une ou plusieurs indications (106A) et du second ensemble d'une ou de plusieurs indications (106B), dans lequel la mémoire (206) stocke des données supplémentaires destinées à être exécutées par l'au moins un processeur (204) qui, lorsqu'elles sont exécutées, amènent en outre l'au moins un processeur (204) à :
déterminer un alignement du champ (102) sur le dispositif chirurgical (104, 214) en fonction d'un alignement du premier ensemble d'une ou plusieurs indications (106A) sur le second ensemble d'une ou plusieurs indications (106B).

8. Système (100, 200) selon la revendication 1, dans lequel la mémoire (206) stocke des instructions supplémentaires destinées à être exécutées par l'au moins un processeur (204) qui, lorsqu'elles sont exécutées, amènent en outre l'au moins un processeur (204) à :
recevoir une image d'une caméra, et
déterminer, à partir de l'image, au moins l'un parmi une obstruction de l'une ou des indications (106) ou un mouvement de l'une ou des indications (106).

9. Système (100, 200) selon la revendication 1, dans lequel l'une ou les indications (106) comprennent au moins une indication optiquement visible et au moins un marqueur infrarouge.

10. Système (100, 200) selon la revendication 1, dans lequel l'une ou les indications (106) sont disposées sur au moins une partie du champ (102).

11. Système (100, 200) selon la revendication 1, dans lequel les informations sont au moins une image obtenue à partir du capteur (212) et la détection de l'une ou des indications (106) comporte le traitement de l'au moins une image à l'aide d'un algorithme de traitement d'image pour détecter l'une ou les indications (106).

12. Système (100, 200) selon la revendication 1, dans lequel l'une ou les indications (106) sont disposées sur le champ (102).

13. Système (100, 200) selon la revendication 1, dans lequel l'une ou les indications (106) sont disposées sur un composant positionné sous le champ (102).
